# EUROPEAN PATENT SPECIFICATION

(11) **EP 3 004 876 B1**
(45) Date of publication and mention of the grant of the patent: **22.07.2020**
(21) Application number: 14807343.0
(22) Date of filing: 02.06.2014
(51) Int. Cl.: G01N 33/543, C40B 30/00, C40B 40/00, C40B 50/00, G01N 35/00, B01L 3/00

(54) **SOURCE CONTROLLED DECODABLE MICROARRAY SYSTEM AND METHODS FOR USE**
QUELLENGESTEUERTES DECODIERBARES MIKROARRAYSYSTEM UND VERFAHREN ZUR VERWENDUNG
SYSTÈME DE MICRORÉSEAU APTE À ÊTRE DÉCODÉ À SOURCE COMMANDÉE ET PROCÉDÉS POUR UTILISATION

(30) Priority: 02.06.2013 US 201313907935
(43) Date of publication of application: 13.04.2016
(73) Proprietor: Ayoxxa Living Health Technologies Pte. Ltd., Singapore 117576 (SG)
(72) Inventor: TRAU, Dieter, Singapore 638404 (SG); SCHMIDT, Andreas, 50829 Köln (DE)
(74) Representative: Michalski Hüttermann & Partner Patentanwälte mbB
(86) International application number: PCT/SG2014/000252
(87) International publication number: WO 2014/196927

(56) References cited:
- US-A1- 2005 049 796
- US-A1- 2010 075 865
- DIANE GERSHON: "Microarrays go mainstream", HHS PUBLIC ACCESS AUTHOR MANUSCRIPT, vol. 1, no. 3, 1 December 2004 (2004-12-01), pages 263-270, XP055340793, GB ISSN: 1548-7091, DOI: 10.1038/nmeth1204-263

## Description

### TECHNICAL FIELD

The present invention generally relates to methods for using source controlled decodable microarray systems.

### BACKGROUND OF THE DISCLOSURE

DNA microarrays are an established technology in genomics and cell biology. Protein and antibody microarrays are a technology also gaining interest and several products are on the market. Potentially, several other types of microarrays such as lipid, carbohydrate, metabolite or drug microarrays could be manufactured for various applications in life sciences, pharmaceutical screening and diagnostics. In the current state of the art, microarrays are manufactured based on three methods: spotting or jet printing, lithographic based methods, and random deposition of microbeads.

In accordance with the first method, a spotting or jet printing based method, microarray forming entities are transferred onto a solid substrate and form a spot at a predefined position. However, all microarrays manufactured by this process are similar to each other. In accordance with the second method, a lithographic based method, monomeric components of the entities forming the microarray are applied one by one to form the polymeric entity. For example, nucleotides are applied to form an oligonucleotide strand, or amino acids are applied to form a peptide. This approach is, however, limited to oligonucleotide and peptide microarrays and, again, all microarrays manufactured by this process are similar to each other.

In accordance with the third method, random deposition of microbeads onto a substrate material, microbeads carrying the microarray forming entities on their surface are applied onto a flat substrate material. The microbeads are fixated onto the substrate to form the array. This method is technically challenging as every single microbead and the entity it carries must be identified. Usually this is done by using a physical identifier on the bead. The physical identifier can be a color code or an oligonucleotide tag. For example, a conventional technique describes the use of a color coding method based on two colors with different intensity ratios to establish a code to distinguish up to several hundred microbead sub-populations. Another conventional technique utilizes oligonucleotide tags (decoding binding ligands) and a further conventional technique describes utilization of a color bar code. In all of these techniques, a sub-population is composed of identical beads with the same entity or binding ligand and the same color code or tag and several sub-populations are mixed to form a bead population. This population of microbeads is then randomly applied to the microarray substrate and the color code or tag enables the identification or decoding of the microarray. In accordance with this third method, each microarray is different from each other yet knowing the code for one microarray allows all microarrays manufactured by this third method to be encoded by a customer. As a consequence, the identity of the beads is known to customers because the color encoding/decoding key is known.

Thus, it can be seen that the manufacturer is unable to restrict the microarray information for all three conventional microbead manufacturing methods, thereby either charging high costs for the microarray sales in order to recoup investment in their development or restricting distribution in order to restrict knowledge of the encoding/decoding key. Yet, microarrays manufactured by all three conventional methods are sold as research tools and are classified as consumables. In use, the consumer will contact the microarray with a sample and perform bioassays on it. After use, the array is disposed and cannot be reused and all analytical or bioassay data is directly known to the consumer at the date of its use. The position of all array forming entity spots on the array is also known to the user enabling instant decoding of the array. For bead microarrays, the consumer also knows all bead positions or can easily decode the array because the decoding code is known.

Dispersion arrays or 3D arrays are another conventional microarray technology where the microbeads are dispersed in a liquid phase and are not assembled onto a solid substrate. The microbeads carry a color code for identification, so this technology also allows a customer to decode all 3D arrays by knowing the color code on one array.

Thus, it can be seen that all conventional microarrays and all existing business models for microarray distribution make the array and all information about the array forming entities, including their position on the microarray and/or color coding of microbeads available to the consumer at the time of purchasing or obtaining the microarray product. What is needed is a method for microarray distribution which can at least partially overcome the present drawbacks and provide an adequate return on the investment to the microarray manufacturer to encourage further research and development of additional microarray entities without disadvantageously restricting distribution of such microassays. Furthermore, other desirable features and characteristics will become apparent from the subsequent detailed description and the appended claims, taken in conjunction with the accompanying drawings and this background of the disclosure.

Article entitled "Microarrays Go Mainstream" by Diane Gershon and appearing in Nature Methods, December 2004, describes a number of microarrays and their properties. A microarray product by Illumina of San Diego California randomly places beads in wells of the microarray and provides decoding information for decoding all of the wells to a customer when the customer purchases the microarray product.

US 2010/075865 A1 describes an encoding/decoding data table unique to the microarray which may be provided to the user wanting to analyse a sample potentially containing one or more target analytes by providing the user with an identifier for access to the encoding/decoding data table.

US 2005/049796 describes that an array is associated with a particular design file using a unique identifier that is physically associated with the array and the design files are typically shipped with arrays that are purchased or may be obtained from a remote location.

### SUMMARY

The invention is defined by the features of the independent claim. Preferred embodiments are defined by the features of the dependent claims.

### BRIEF DESCRIPTION OF THE DRAWINGS

The accompanying figures, where like reference numerals refer to identical or functionally similar elements throughout the separate methods and views and which together with the detailed description below are incorporated in and form part of the specification and serve to illustrate various embodiments and to explain various principles and advantages in accordance with a present invention.
FIG. 1, comprising FIGs. 1A and 1B, illustrates micrographs of microbeads of a microassay product, where FIG. 1A illustrates an atomic force micrograph of the microassay product having the microbeads assembled onto a glass substrate, while FIG. 1B illustrates a scanning electron microscope (SEM) micrograph of the microarray product having the microbeads fixated onto a substrate having three-dimensional microwells in accordance with a present embodiment.
FIG. 2 illustrates a process for producing the microarray product of FIG. 1B in accordance with the present embodiment
FIG. 3 illustrates a first method for manufacture, distribution and decoding the microarray product of FIG. 1B in accordance with the present embodiment.
And FIG. 4 illustrates a second method for manufacture, distribution and decoding the microarray of FIG. 1B in accordance with the present embodiment.

Skilled artisans will appreciate that steps and elements in the figures are illustrated for simplicity and clarity and have not necessarily been depicted to scale. For example, the timing between some of the elements in the flowcharts may be shrunk in respect to timing between other elements to help to improve understanding of the present embodiments.

### DETAILED DESCRIPTION

The following detailed description is merely exemplary in nature and is not intended to limit the invention or the application and uses of the invention. Furthermore, there is no intention to be bound by any theory presented in the preceding background of the invention or the following detailed description. It is the intent to present methods for manufacture and distribution of microarray products while controlling the decoding of the microarray products.

### DEFINITIONS

The term "microarray product" means any DNA, oligonucleotide, protein, antibody or peptide microarray, or in general a microarray formed from "chemical entities". The chemical entities are sourced from "providers" and/or are made available by the manufacturer to manufacture the microarray product. The microarray product can be manufactured by all known methods such as by spotting, by contact printing, jet printing, microcontact printing, dip pen lithography, using lithographic methods or by the random deposition of nanobeads and/or microbeads onto a substrate. The term "microarray product" herein also includes so called suspension arrays, liquid arrays, or 3-D arrays. In general the term "microarray product" in the context of this disclosure includes an analytical device and/or reagent kit for multiplex analysis with a typical plex of more than three.
The term "chemical entity" or "chemical entities" means any material, molecule or chemical to form a microarray product. The chemical entity can interact with an analyte in the sample to cause a measurable signal. Typically, but not limiting the definition within the present disclosure, chemical entities are DNA, RNA, nucleic acids, oligonucleotide, proteins, antibodies, enzymes, ribozymes, peptides, cytokines, cell lysates, aptamers, polymers and small molecular weight chemical entities such as, but not limited to, drugs, enzyme substrates, hormones, lipids, carbohydrates, antibiotics, growth factors, toxins, metabolites, secondary metabolites, libraries of synthetic organic compounds and libraries of natural compounds. As set out in the definition of microassay product above the microarray product in accordance with the present embodiment is formed of more than three chemical entities.
The term "consumer" means the end user of the microarray product. The end user of the microarray product typically contacts the microarray product with his sample and performs bioassays or decodes the analytical information. Without limitation, typical end users are customers, researchers, scientists, engineers, clinicians, students, military agencies, nurses, any staff involved in research and development work or food, drug or environmental testing or medical diagnostics, or any service providers or testing labs. Typically, end users are skilled in the art of bioassays or chemical analysis. End users may work at various institutions such as universities, research institutes, industry research labs, health organizations, hospitals, military organizations, environmental organizations, clinics, insurance companies. Airlines or other industries. However, selected microarray products may be used by a layman at home or in the workplace.
The term "obtaining" and its grammatical variants such as "obtained", "obtain" and "obtains", means to come into possession of a physical microarray product or into possession of a decoding information data package with the intention to use them to perform bioassays or to decode the analytical information. The microarray product can be obtained by known existing channels such as over the counter purchase or by delivery. The decoding information data package can be obtained in various ways, because it consists of data which can be transmitted from the manufacturer to the customer. The transmission of such data can be performed wirelessly or via a fixed data transmission line. Without limitation, the transmission is typically performed via email, over the internet, over a telephone line, via a data storage media, via printed media or via software. A sending and/or a receiving device for the transmission are typically, but not limited to, a computer, a portable computer, a hand phone or an analytical instrument. The physical microarray product and the logical decoding information package or decoding key may be linked to each other by the use of a request key as described herein. A consumer can send or execute a request key for selected analytes to the manufacturer to request or obtain the decoding key. The physical microarray product and/or the decoding information data package can be obtained from the manufacturer or from a distributor of such or via any other channels.
The term "bioassays" or "bioassay" means the interaction of the sample with at least one "chemical entity" on the microarray product to determine analytes present in the sample. The interaction can be a binding reaction, a chemical reaction, an enzymatic reaction or a physical interaction. Typically, an analyte in the sample is interacting with a specific chemical entity of the microarray product to cause a measurable signal. An analyte can also interact with more than one chemical entity. The signal provides qualitative or quantitative information about the presence of the analyte in the sample. The microarray product in accordance with the present embodiment can detect three or more analytes in parallel, this process being termed "multiplexing". The term "bioassay" includes, but is not limited to, antibody assays, sandwich immune assays, enzymatic assays, hybridization assays, receptor ligand binding assays, FRET assays, fluorescent assays, luminescent assays, radio isotope based assays, quantum dot assays, quenching assays, microbial assays, enzymatic assays, cell assays, inhibition assays, activation assays, chelating assays, toxicity assays, gene expression assays, home tests and mixtures thereof. The term "bioassay" also includes screening assays to screen for a compound in the sample or to screen for a chemical entity on the microarray product.
The term "decoding information data package" means information that is necessary to decode the microarray product thereby correlating signals from the bioassays with respective analytes in the sample. The decoding information data package is a decoding key for the microarray product and, when obtained by a consumer, may contain all information to correlate all bioassay signals to all analytes or may contain only partial information. The information for fully decoding the microarray product may be split into more than one decoding information data package. For example, the decoding information data package may be split into a decoding information data package exclusively for control measurements and at least one other decoding information data package exclusively for analytical data. The decoding information data package may not be a physical product as it only consists of data and therefore can be conveniently and fast delivered to the customer in accordance with any of a number of delivery schemes. The decoding information data package can be made available to the customer as a data file of any format, as software, as data storage media, or as printed media. It must be noted that the customer can not make use of the microarray product to analyze a sample without the decoding information data package. The customer can perform the physical process of the bioassay but is not able to correlate the bioassay signals to the respective analytes without the decoding information data package. The decoding information data package can be obtained by the customer before, at the same time, or after obtaining the microarray product. Alternatively, the decoding data package and the microarray product can be purchased by two different entities.
The term "manufacturer" in general means the producer of the microarray product. In the context of this disclosure, the term "manufacturer" also includes the distributor of the microarray product or any party from which the microarray product is obtained by the consumer. Typically, the party from which the microarray product is obtained also provides the "decoding information data package" to the consumer. However, in certain cases the consumer can obtain the physical microarray product from the producer of the microarray and the decoding information data package from a distributor or vice versa. It should be pointed out that in the context of the present embodiment the events of obtaining the physical microarray product and the event of obtaining the decoding information data package are, at least for one decoding information data package, two distinct events in time.
The term "provider" means a person, a scientist, an engineer, a medical practitioner, a company, an institute, a university, a student, a hospital, a clinician, a doctor or an institution that provides at least one chemical entity to the manufacturer to form a microarray product. The provider may provide the at least one chemical entity to the manufacturer for free, for a fee or in consideration of royalties. The chemical entities forming a microarray product can be all provided by the manufacturer or all provided by at least one provider or by a mixture thereof. In the context of the current disclosure the manufacturer is also a provider.
The term "payment channel" means a method to pay a fee by the customer or end user to the manufacturer or distributor or to make royalty payments by the manufacturer to a provider and should include but is not limited to payment over the counter, payment by bank transfer, payment via the internet, credit/debit card payment, check payment, prepaid card payment, pay pal payment, payment through a customer account, prepaid payment and/or mixtures thereof.
The term "web based platform" means a web based software to facilitate interaction between the manufacturer, consumers and providers. Typically, the web based platform is provided by the manufacturer or a distributor. The web based platform may provide any or all of the following functions: a user interface for customers; a customer account; a data storage for customers to store analytical data obtained by using the microarray product; payment channels; bioinformatic tools for customers to analyze data; networking functions to network or contact other customers or to share data with other customers or to invite any party to access the data; a user interface for providers; and a revenue sharing function for providers.
The term "data exchange platform" means any platform for exchanging a "decoding information data package" or any other type of data relating to the microarray product, its distribution, its decoding or its use. A "data exchange platform" may include a web based platform or may include data transmission by email, internet, or over a telephone line. A "data exchange platform" may also include hardware data transfer modes such as data storage media or printed media or software data transfer.
The term "request key" means information or a code to link a physical microarray product with the respective decoding information data package. One physical microarray product may have several request keys. Request keys are made available to the consumer to request decoding information data packages. Execution of a request key means that the key is sent to request the decoding key followed by the decoding key being sent to the consumer. For the execution of the request key, a consumer may pay a fee. The request key links the physical microarray product with the non-physical data and/or with decoding information data packages. A request key may be made available in a secured way or encrypted to limit access to decoding information. The identity of the request key holders may be known and a request key holder may need to authenticate himself to the manufacturer as an authorized request key holder to execute the request key. A request key may also be used in shared revenue models. In accordance with one variant of the present embodiment, a consumer executes the request key for a chemical entity on the microarray product. In case the chemical entity was made available by a provider, the execution of the request key initiates a royalty payment to the provider.

The methods, systems and business models in accordance with variants of the present embodiment all employ a microarray product which consists of two components. The first component is a physical microarray product necessary to perform a bioassay. The second component is a decoding information data package to decode the microarray product.

### DESCRIPTION OF EMBODIMENTS

A fundamental aspect of the present embodiments is that a consumer will always need both a microarray product which is a physical entity and a decoding information data package which is a logical entity to analyze a sample. A further aspect is that the decoding information for a given array may be split in several decoding information data packages and that at least one data package is made available to the consumer at a discrete time period different than the time the consumer obtains the physical microarray product. As an example not part of the present invention a method is provided to use a microarray product by a consumer including the steps of obtaining the microarray product by the consumer, contacting the microarray product with a sample and performing bioassays by the consumer, and obtaining a from the manufacturer of the microarray product to enable the consumer to decoding information data package correlate the bioassay signals from the microarray bioassays with the respective analyte, the decoding information data package obtained at a time discrete and different than the time the consumer obtains the microarray product.

The method and system in accordance with this example has several advantages over the state of the art. The manufacturer can produce a generic product which contains several sub-groups of analytes. Depending on which decoding information data package is made available to the consumer different analytes can be measured. The manufacturer also has full control over the microarray product and the decoding of the bioassay signals of the microarray product. By providing only selected decoding information to a customer, the manufacturer can literately switch on and off selected analytes. This feature of this example enables a number of new business models for microarray products with value propositions for both the manufacturer and the customer. The methods, business models and value propositions are outlined hereinbelow.

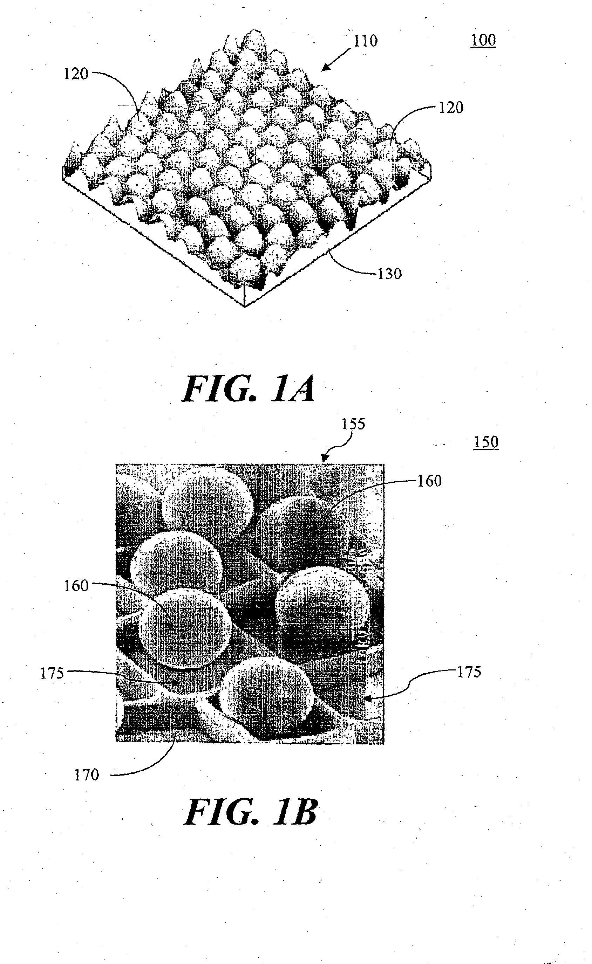

In accordance with this example, manufacture of the microassay product should make it virtually impossible for a customer to decode the microarray without data provided by the manufacturer for every individual microarray. An example of such microarray products are depicted in FIGs. 1A and 1B. Referring to FIG. 1A, an atomic force micrograph 100 illustrates a microarray product 110 having microbeads 120 assembled onto a glass substrate 130 in accordance with one embodiment. The glass substrate 130 is positively charged by its coating with poly (alylamine hydrochlorid). The microbeads 120 carry negative charges and are fixated to the substrate 130 by coulomb forces. Referring to FIG. 1B, a scanning electron microscope (SEM) micrograph 150 depicts a microassay product 155 in accordance with another example wherein polystyrol microbeads 160 are fixated onto a substrate 170 having three-dimensional structures of microwells 175.

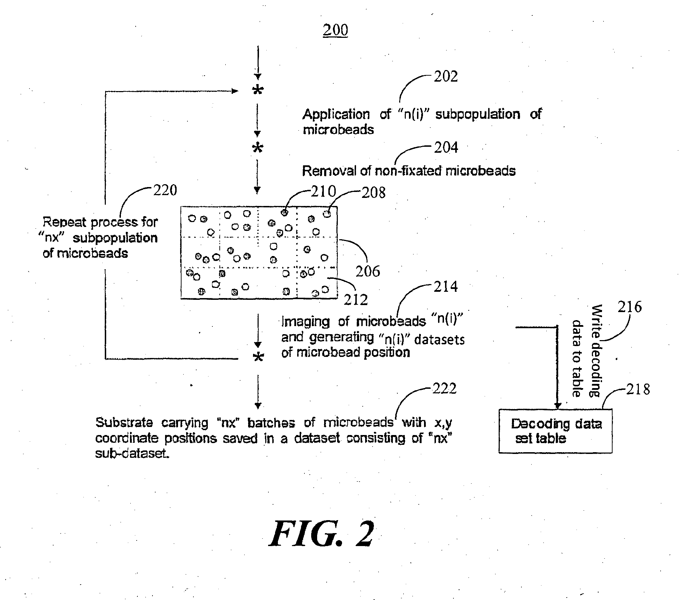

Referring to FIG. 2, a process flow diagram 200 depicts steps for a portion of a process to produce either the microarray product 110 or the microarray product 155 in accordance with an example not part of the present invention. During the process of FIG. 2, a substrate is treated 202 with a subpopulation "n(i)" of microbeads that includes an active agent capable of binding to at least one target analyte on the surface of the microbead.

After the treatment 202, non-fixated microbeads are separated or washed off 204 the substrate. The diagram 206 depicts microbeads 208 of target analytes previously fixated to the substrate and microbeads 210 of subpopulation "n(i)" on the substrate 212 after the non-fixated microbeads are separated 204. The attached microbeads 210 of subpopulation "n(i)" are then imaged 214 and their coordinate positions relative to a fiducial on the substrate 212 are stored in a data set related to the batch number "n(i)" of the microbeads 210 and a chip number related to the substrate 212, the data set written 216 to a decoding data set table 218.

The process 202, 204, 214, 216 is repeated sequentially 220 for "nx" subpopulations of microbeads until the desired number ("nx") of microbead subpopulations have been applied onto the substrate. At the end of the process, a substrate 222 carrying "nx" batches of microbeads and an encoding/decoding data table 218 containing all microbead positions for each batch will result.

The present process 200 describes one approach to form a random bead microarray 222 sequentially in discrete time periods of each other on a flat substrate material 212 where after each random deposition 202, 204 an image 214 of the microarray is registered. By comparing the sequential images with each other the position of every single microbead 208, 210 is decoded and known. In accordance with the present embodiment, the microbeads 208, 210 used in the process are identical in appearance. Because the microbeads 208, 210 do not have any physical identifier and every array is different from each other, it is impossible for the customer or any other party to decode the microarray 222. Only the manufacturer who holds the decoding code 218 as the x and y position of each single microbead 208, 210 is able to decode the microarray 222.

While FIG. 2 discloses one process for making source decodable microarrays, those skilled in the art will realize that other processes or alterations, additions or changes to the process 200 may be developed in keeping with the goals of the present embodiments. This, the present embodiments encompass a method to use a system and a microarray and methods for using such microarrays in which the consumer obtains a physical microarray and at least one logical decoding information data package at discretely different times.

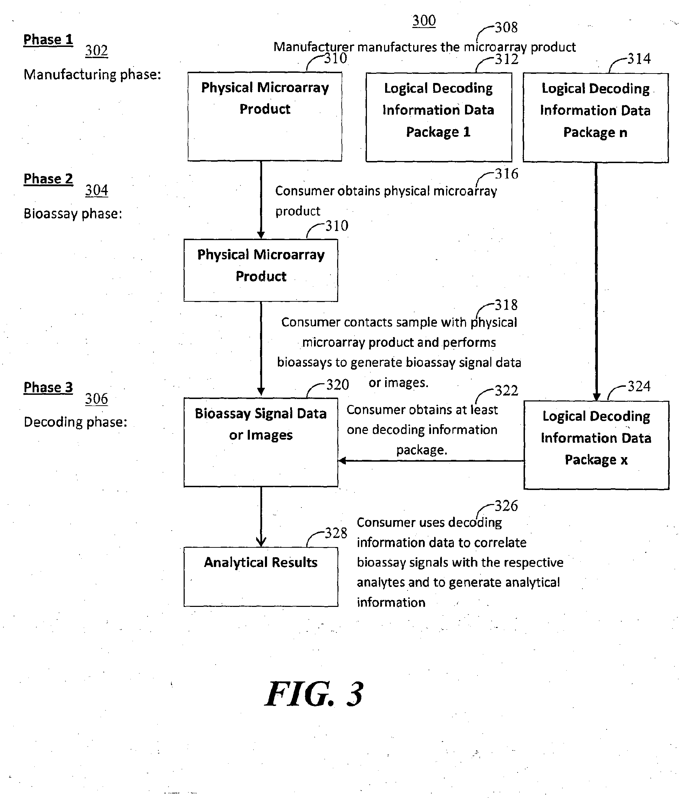

Referring to FIG. 3, a diagram 300 depicts a business model for use of a microarray product 222 in accordance with a present embodiment. The entire method of using the microarray product 222 in accordance with example is divided into three phases 302, 304, 306. In phase 302, also termed the "manufacturing phase", the microarray product 222 is manufactured 308 and thereby the physical microarray product 310 is created. The physical microarray product 310 should preferably be formed by at least three chemical entities. In parallel to the manufacturing 308, a logical decoding information data package 218 is also created. Thus, the entire microarray product always consists of two components: the physical microarray 310 and the decoding information 218. The logical decoding information data package can be split into more than one logical decoding information data packages 312, 314.

In phase 304, also termed the "bioassay phase", a consumer obtains 316 the physical microarray product 310 and contacts 318 the physical microarray product 310 with a sample to perform bioassays. On a typical microarray product 310, a large number of bioassays are performed, termed multiplexing. In accordance with the present example, the physical microassay product 310 should preferably permit at least three bioassays to be performed on the deposited chemical entities. More preferably, the physical microassay product 310 should allow more than ten bioassays. High multiplex products 310 in accordance with the present example may permit up to one hundred bioassays, and ultra high multiplex microarray products 310 may allow ten thousand or more bioassays. During each bioassay, a specific chemical entity on the microarray 310, e.g. an antibody is interacting with an analyte such as an antigen and a bioassay signal is generated. In selected cases, the bioassay may include several incubation washing steps with additional reagents to generate the bioassay signal. In all cases, all specific chemical entities on the microarray product 310 will interact with its respective analyte if the analyte is present in the sample. Therefore, all bioassays are performed by the consumer in a single bioassay protocol 318. The signals from all specific chemical entities are recorded by the consumer 320. During phase 304, the consumer is not able to correlate the signals with the respective analytes.

In phase 306, also termed the "decoding phase", the consumer obtains 322 at least one logical decoding information data package "x" 324. The at least one logical decoding information data package 324 will enable the consumer to correlate the physical signals 320 from the microarray 310 with the logical analyte information and thereby determine 326 the presence of analytes in the sample (i.e., the analytical results 328).

Optionally, the consumer may obtain 322 additional logical decoding information data packages 324 at any later point of time to decode 326 more analytical data 328. Preferably, the microarray product 310 includes at least two logical decoding information data packages 312, 314. Conveniently, the consumer may obtain 322 the logical decoding information data package 324 over the internet. Advantageously, no further bioassays are necessary to be performed by the consumer. In accordance with the present example, such further packages 324 are released in consideration for a fee, allowing the manufacturer to advantageously receive further income streams after the initial product 310 was made available to the consumer.

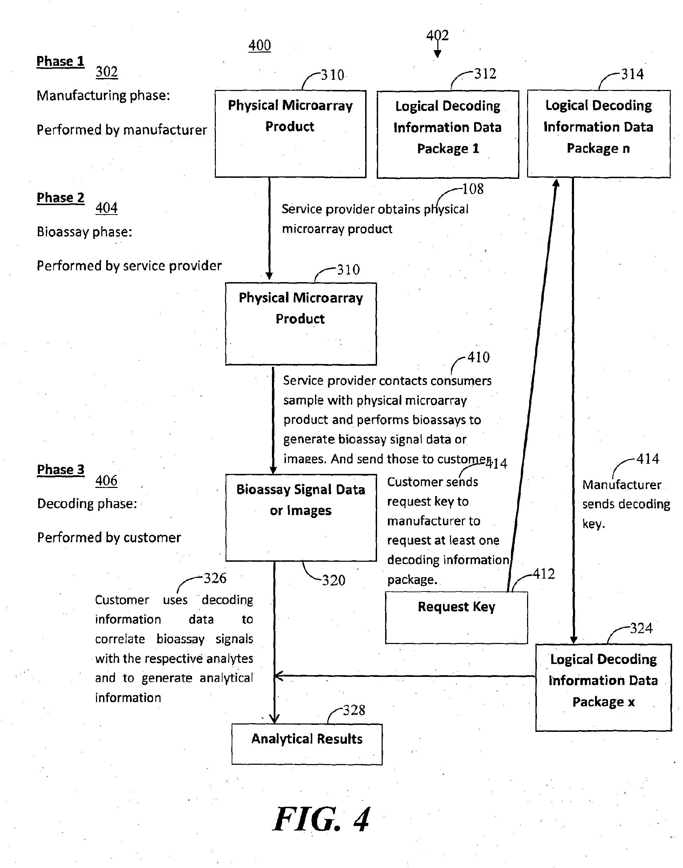

Referring to FIG. 4, a second process 402 is illustrated in the process flow diagram 400, in which at phase 404 the microarray is obtained 408 and testing is performed 410 by a first party and the analytical test results (phase 406) are only made available by a second party. This second process 402 relies on the use of a request key 412 requested 414 by the second party and a decoding key received 414 in response to the request key 412. In particular, the process 402 enables a service provider to perform the test 410 but not come into possession of the analytical data 328, e.g. confidential patient data. In general, the process 402 provides a non-limiting example of the use of a request key 412 and a decoding key 414 in accordance with a present embodiment.

Thus it can be seen that in preferred examples of the method and system of using a microarray, the customer or consumer obtains at least one decoding information data package 324 at a discrete time period different than the time the consumer or service provider obtains the physical microarray product 310. The consumer may obtain a first decoding information data package (e.g., package 312) together with the physical microarray product 310 and performs the bioassay and the decoding. At any later time, the consumer may obtain at least one more decoding information data package 324 to decode additional information on the same microarray product 310. This is advantageously enabled without performing a new bioassay. Conveniently, the consumer obtains the additional decoding information data packages 324 via the internet for a fee. In this manner, the consumer can buy additional analyte information at any later date and the manufacturer receives further income after the physical microarray product 310 is sold.

In another example nor part of the present invention, the manufacturer may manufacture a generic microarray product 310. A generic microarray product 310 in the context of this embodiment is a microarray that contains more than one panel of analytes. For example, five different panels of twenty analytes each for various different applications may be provided in the physical microarray product 310. The consumer then obtains the generic microarray product 310 first and decides on the panel of his interest. The consumer may then obtain the respective encoding information for the panel of his interest.

The generic microarray product has advantages for the manufacturer. Those skilled in the art will realize that the manufacturer can manufacture a generic microarray in larger quantities for different groups of customers thereby reducing the manufacturing cost per microarray. The customer also benefits from the generic microarray product. For example, the customer can use the same microarray product for different applications decided by the customer after purchase of the microarray product, thereby providing more flexibility to the customer.

In accordance with another example not part of the present invention, the customer may obtain a number of the physical microarray products from the manufacturer for free or for a nominal fee. Only in the event that the customer performs an experiment, then he may obtain at least one decoding information data package for a fee. In the event, the customer uses the microarray product but the experiment fails the customer may not obtain a decoding information data package thereby saving cost to the customer. In this example, the major payment is done at the point of performing the bioassay but not at the time of obtaining the physical microarray product. Because it is impossible for the customer to decode the microarray, he has no other choice than to obtain the data package for a fee from the manufacturer. This represents a "pay on demand" business model and is novel in the field of microassays.

In accordance with an embodiment of the present invention, a first entity of a customer obtains a number of physical microarray products from the manufacturer and perform bioassays and tests with the microarray product. This first entity of the customer does not have access to any decoding information data packages. This first entity of the customer may hold a decoding information data package only for control measurements on the microarray product to determine if the assay was performed correctly, but not for analytical data of analytes on the microassay product. Typical control measurements include blanks, calibrations, and bead intensity measurements for calibration and normalization. This first entity of the customer transmits the undecoded data or image files to a second entity of the customer. Only the second entity of the customer can access the decoding information data package to decode the analytical, biological or chemical test data. Advantageously, this can be used for patient data, health care systems, criminal investigation data, food and drug testing, military testing and other confidential or sensitive data testing where a testing laboratory can perform tests but the knowledge of test results must be made available only to a limited group of people excluding the performer of the biological or chemical test. In this business model the first entity of the customer is a service provider to the second entity of the customer. The first entity of the customer can provide the service and ensure the quality of analytical data through the decoding information data package exclusive for control measurements but has no access to analytical data. The second entity of the customer is a consumer for the first entity of the customer and has the ability to decode the analytical data. The first entity of the customer and the second entity of the customer may obtain the respective decoding information data package from the manufacturer by using a request key or code. The decoding information data package is then sent to the customer after the manufacturer confirms the identity of the customer entity. The decoding information data package may be sent as an encrypted file. However, if an encrypted microarray product is used, encryption of the decoding information data package may not be an absolute requirement. As discussed above, the method of using a microarray in accordance with the present embodiment delinks the analytical information from the control information on the microarray product. As a result, the method allows a service provider to perform bioassays and tests without knowing the analytical information. Another party then receives the undecoded data or image files and, by obtaining the decoding information data package from the manufacturer, can decode the microarray and obtain the analytical information.

Another example not part of the present invention is related to the restriction of data to consumers or providers. The decoding information data package may be split into a decoding information data package exclusively for control measurements and at least one other decoding information data package exclusively for analytical data. Advantageously for a microarray product produced in accordance with the method of FIG. 2, the manufacturer holds the decoding information data package or decoding key for the microarray product. The decoding key can be made available to a consumer or the consumer may request the decoding key. The manufacturer may also require identification and authentication of the customer before releasing the decoding key.

In a revenue sharing business model in accordance with a further example not part of the present invention, at least one chemical entity to form the microarray is provided by a provider other than the manufacturer. In the event the customer obtains a decoding information data package that contains chemical entities provided by at least one such provider, the fee paid by the customer for the said decoding information data package is shared with these providers. Typically, the manufacturer and the at least one provider have entered into an agreement that specifies the term of the revenue sharing business model (e.g., royalties due such at least one provider in response to use of such at least one provider's proprietary chemical entities). When a request key is used by the consumer to request the decoding data, execution of the request key triggers the event of the royalty payment.

The method of using a microarray product and business model in accordance with the embodiment and examples discussed herein has distinct advantages over conventional microarray and bioassay product. In current business models, the manufacturer has no knowledge if and when its products are used by a consumer after delivery to the consumer. This has several disadvantages. The manufacturer has no information if the customer requires new products, no information if the product was used, no information about the quality of data obtained with the product and no information on the most preferred analytes. In contrast, the methods and business model of embodiments discussed herein employ the use of a non physical request key and decoding information packages necessary to use the physical microarray product.

In preferred embodiments, a customer first obtains the physical microarray product. In the event of performing a bioassay using the microarray product, the customer will execute a request key to request the decoding information package to decode the analytical information. This unique feature of the methods and business models of the present embodiments provides real time information to the manufacturer if and when a consumer performs a bioassay with the manufacturer's microarray product. Advantageously, this information may be used by the manufacturer in different ways to serve the customer and promote the manufacturer's business. A non-exclusive list of such services and promotions are: (a) the manufacturer may send information or publications to the customer related to analytes requested by executing the request key, (b) the manufacturer may provide bioinformatics services to analytes requested by execution of the request key, (c) the manufacturer may deliver technical notes or tips to the consumer as he has information about the quality of the bioassays performed by the consumer, (d) the manufacturer may deliver microarray products to the consumer as he has information that the consumers microarray products are used up, and (e) the manufacturer will gain valuable market information as he has information about the most requested analytes by customers. Currently, only the methods of using a microarray product and business models in accordance with present embodiments discussed herein will provide such real time information to the manufacturer, if, when and how the customer is using a microarray product enabled by the features of the request key and the encoding information data table of the present invention. Advantageously, the exchange of information such as request keys, decoding information, technical information, bioinformatics information and marketing information is done via a web based platform and/or the internet and/or an application or software run on a mobile device.

In accordance with another method of using a microarray and business model, at least one analyte is selectively "switched" on or off for microarrays sold in different markets. The microarray in accordance with this embodiment may be a diagnostic product and a certain analyte may not be approved or may be approved as a diagnostic marker in a certain market or territory or for a particular diagnostic application and is accordingly "switched" on or off by providing or not providing the respective encoding information data to the customer, consumer or medical practitioner or staff. The business model in accordance with this embodiment has the following advantages: (a) only one generic product is manufactured to save cost, (b) a diagnostic marker or analyte can be "switched" on or off at demand without manufacturing a new product, and (c) a direct response to legal changes in a territory or market is possible. Such a case could be the approval of a biomarker in a territory and the biomarker information can be made available immediately by providing the respective decoding information data.

In general, the methods and systems of using a microarray and business models in accordance with embodiments discussed herein give the manufacturer full control over the microarray product. To make sure customers use the manufacturer's reagents, the reagents may be bundled with the encoding information data packages.

The methods of using a microarray and business model in accordance with embodiments discussed herein may also be further facilitated by a web based platform provided by the manufacturer and acting as a data exchange platform. The web based platform may provide the following functions: (a) a user interface for customers, (b) a customer account, (c) a data storage for customers to store analytical data obtained by using the microarray product, (d) payment channels, (e) bioinformatic tools for customers to analyze data, (f) networking functions to network or contact other customers or to share data with other customers or to invite any party to access the data, (g) a user interface for providers, (h) a revenue sharing function for providers, (i) a quality assurance system for the manufacturer, (j) a market research and quality assurance system, (k) a system to avoid product counterfeiting of reagents and microarrays, (1) an online shop, (m) an automated ordering system, (n) a discussion forum, (o) a database, and/or (p) a search function. The internet based user interface or user platform enabling present embodiments of the web based platform may include: (a) a gaming function, (b) a linking function to social media platforms, (c) a lottery function, or (d) a purchase history function.

To improve the credibility of published scientific data, the internet based user interface or user platform may also include a referencing function to link published scientific data with the original raw data set or with a selected set of data originating from the use of the microarray product. This will allow the reader of a scientific publication to retrieve data sets from the internet by using the reference linked to the data originated from the use of the microarray product. This reference service function of the present invention will greatly benefit the scientific community and add credibility to the data of a user of the microarray product.

### EXAMPLES

### Example 1 (not part of the present invention):

In a typical scenario, the customer is a researcher and obtains the microarray product directly from the manufacturer. The microarray product in this scenario should contain a number of analyte groups, say analyte group 1 to group n. Typically such analyte groups are related to answering a certain research question, for example analyte group 1+x contains cytokines related to cell differentiation, analyte group 2+x contains cytokines related to adoptosis, and analyte group 3+x contains other biomarkers related to various other cell states. Together with the microarray product the customer obtains the decoding information data package for only one analyte group 1+x. The customer performs bioassays and can analyze the analytes of group 1+x. However, physically all bioassay reactions are already performed and the customer has measured all bioassay signals. In a typical scenario, the customer may be interested in additional analytes not present in the initial group 1+x. The customer can then obtain additional decoding information data packages at any later period to decode the remaining groups 2+x and 3+x. Conveniently, the customer obtains such additional data packages via the internet. In addition, the remaining data sets may be released to the customer in consideration of a fee. Typically the fee is paid through the internet via a credit card or other payment instruments or is deducted from an existing account the customer holds with the manufacturer.

### Example 2 (not part of the present invention):

In another typical scenario, the customer is a clinician at an emergency care unit of a hospital and the microarray product is obtained directly from the manufacturer without obtaining any decoding information data packages. The microarray product in this scenario should contain a number of analyte groups related to infectious diseases, say analyte group one to group four. In this example, group one is related to bacterial infections, group two to viral infections, group three to parasitic infections, and group four to a number of rare infections.

A patient may be admitted to the emergency care unit and the clinician uses the microarray product for clinical diagnostics with the patient's sample. The clinician suspects a viral infection and therefore obtains the decoding information data package for group two. In a first possible scenario, a viral infection is confirmed, and the customer need only pay a fee for group two. In another possible scenario, the viral infection is not confirmed and the customer may decide to obtain the additional data package for group one by paying an additional fee.

This Example 2 illustrates several advantages of present embodiments over the state of the art. First, all bioassays are already performed during the first testing. To add additional groups does not cause a delay and no additional physical assays are necessary. The addition of additional groups is performed in seconds to minutes, rather than hours or days. The addition of additional groups could be based on the decision of the clinician or could be automated using an expert system. Second, the person performing the physical testing with the patient sample under no circumstances will know patient data. The access to the decoding information data package(s) can be restricted to the clinician only to guarantee the privacy of the patient. Third, the method for partial use of the analytical information on the microarray saves cost for the customer and enables the manufacturer to manufacture a generic product at a larger scale to also save manufacturer's costs.

### Example 3 (not part of the present invention) :

In a royalty sharing business model, at least one chemical entity forming the microarray product is provided by a provider. In some cases, all chemical entities forming the microarray are provided by one or more providers. A customer may contact said microarray product with a sample and perform bioassays. The customer may observe signals generated by certain bioassays but, since the customer has not obtained any decoding information data package, the customer is not able to correlate the signals with the respective analytes or correlate the signals with the identity of the chemical entity on the microarray that gave rise to the signal. The customer may request at least one decoding information data package from the manufacturer to correlate at least one signal with the respective analyte or identify the chemical entity on the microarray that gave rise to the signal. The decoding information data package is released to the customer for a fee or for royalties and, in this royalty sharing business model, the fee or royalty is shared between the manufacturer and the provider of the said chemical entity that gave rise to the signal. A fee or royalty is then paid by the consumer to the manufacturer for the information on the analyte or to use the now identified chemical entity for an application by the customer. The exact terms and amount of the fee or royalty may be specified in a royalty agreement between the customer and the manufacturer. The royalty agreement may be part of the microarray product or may be made between the customer and the manufacturer before, at the time of, or after the customer obtains the microarray product. In a typical scenario of this Example 3, the customer is screening for a chemical entity or drug for a specified application or treatment and the royalty sharing model only comes into effect after identifying such chemical entity or drug.

**Example 4:** In a service provider business model, the end user (in the following called "customer") of the data and the end user of the physical microarray product (in the following called "service provider") are two different parties. The customer or service provider orders a microarray product from the manufacturer. The microarray product is delivered to the service provider. The decoding keys for control measurements are made available to the service provider and the decoding key for analytes is made available to the consumer. The consumer sends a sample to the service provider and the sample is tested. The service provider sends the undecoded data to the manufacturer and or consumer for decoding of the analytical information. The decoded analytical information thus is only available to the customer and not the service provider.

### Example 5 (not part of the present invention):

In another business model example, the microarray product contains a panel of analytes to test (e.g. patient blood samples). In addition, the microarray product contains at least one more chemical entity. This additional chemical entity is provided by the manufacturer or a provider. The end user of the microarray product conducts testing but only has access to the panel analytical data. The analytical data of the additional chemical entity is made exclusively available to another party for a fee. The other party may be the provider or a pharmaceutical company. In this "use for a fee" business model, a pharmaceutical company provides chemical entities and analytical data related to the chemical entities is sold to the pharmaceutical company. To hold up ethical and moral standards, patient consents must be obtained and other legal requirements must be followed. In this business model, the microarray product is used as a test bed by a pharmaceutical company to acquire analytical data for drug or biomarker development. In a variation of this business model, a consumer may give access to his analytical data to a third party for a fee. The third party may be a pharmaceutical company or a scientist and the manufacturer may facilitate such business models with a web based platform.

### Example 6 (not part of the present invention):

In another business model, the microarray product is a diagnostic product to test for infectious diseases. The microarray diagnostic product may be used around the world and data decoding is performed over a centralized server. In case an infection was diagnosed or not diagnosed, anonymonized data is published on a centralized server including identification of the infectious disease, the time, date and location of the testing, and anonymonized patient data such as sex, age, race, body weight and height. For the benefit of local and world populations, this business model should be on a nonprofit basis and information could be made globally available in real time for epidemic and pandemic monitoring. As a variation of this business model, the end user or hospital or clinic providing the analytical data to the centralized server could receive a fee from the nonprofit organization. This business model is beneficial for developing countries to reduce the cost of its health care system or the cost of screening the population for infectious diseases. By using a microarray product manufactured in accordance with FIG. 2 and using the request key and decoding key of FIG. 4 no fraudulent activities are possible. The business model also benefits the first world as epidemics can be detected before a pandemic evolves. It will be appreciated by those skilled in the art that the business model outlined above may create a major impact on global health. It benefits local and global populations in developing and first world countries and offers an incentive and cost reduction for health systems. Most advantageously, fraud is almost made impossible by using features in accordance with present embodiments disclosed herein.

Thus, it can be seen that the present embodiments and examples provide methods, systems and business models for microarray product distribution which can at least partially overcome the drawbacks of present systems, methods and business models and provide an adequate return on the investment to the microarray product manufacturer to encourage further research and development of additional microarray products without disadvantageously restricting distribution of such microassay products. In addition, it provides methods and systems which are beneficial to both consumers and manufacturers by separating decoding of bioassay signals from microarray products from the provision of such microarray products allowing more generic microarray products to be manufactured and a plethora of income stream models based upon requesting and providing decoding information data packages separate from the microassays. While exemplary embodiments have been presented in the foregoing detailed description of the invention, it should be appreciated that a vast number of variations exist.

It should further be appreciated that the exemplary embodiments are only examples, and are not intended to limit the scope, applicability, operation, or configuration of the invention in any way. Rather, the foregoing detailed description will provide those skilled in the art with a convenient road map for implementing exemplary embodiments of the invention, it being understood that various changes may be made in the function and arrangement of steps, business models, and methods of use described in the exemplary embodiments without departing from the scope of the invention as set forth in the appended claims.

## Claims

1. A method for using a microarray product, the method comprising:
obtaining a microarray product (310);
performing a bioassay by a first customer using said microarray product (310);
obtaining by said first customer from a manufacturer of the microarray product (310) a first decoding information data package (324) for control measurements on the microarray product (310) to determine if the assay was performed correctly but not for analytical data of analytes on the microarray product (310); and
obtaining, by a second customer, from a manufacturer of the microarray product (310) a second decoding information data package (324) for analytical data of analytes on the microarray product (310).

2. The method in accordance with Claim 1 wherein the first customer is an analytical lab or service lab, and wherein the second customer is a medical practitioner.

3. The method in accordance with Claim 1 wherein the first decoding information data package for control measurements on the microarray product (310) enables control measurements including blanks, calibrations and bead intensity measurements for calibration and normalization, and wherein the second decoding information data package (324) decodes patient-related analytes.

## Patentansprüche

1. Verfahren zum Verwenden eines Microarray-Produkts, wobei das Verfahren die Schritte aufweist:
Erhalten eines Microarray-Produkts (310);
Ausführen eines Bioassays durch einen ersten Kunden unter Verwendung des Microarray-Produkts (310);
Erhalten eines ersten Decodierungsinformationsdatenpakets (324) für Kontrollmessungen bezüglich des Microarray-Produkts (310) durch den ersten Kunden von einem Hersteller des Microarray-Produkts (310), um zu bestimmen, ob der Assay korrekt ausgeführt wurde, jedoch nicht für analytische Daten von Analyten bezüglich des Microarray-Produkts (310); und
Erhalten eines zweiten Decodierungsinformationsdatenpakets (324) für analytische Daten von Analyten bezüglich des Microarray-Produkts (310) durch einen zweiten Kunden von einem Hersteller des Microarray-Produkts (310).

2. Verfahren nach Anspruch 1, wobei der erste Kunde ein analytisches Labor oder ein Servicelabor ist und wobei der zweite Kunde ein Arzt ist.

3. Verfahren nach Anspruch 1, wobei das erste Decodierungsinformationsdatenpaket für Kontrollmessungen bezüglich des Microarray-Produkts (310) Kontrollmessungen einschließlich Blindtests, Kalibrierungen und Bead-Intensitätsmessungen zur Kalibrierung und Normalisierung ermöglicht, und wobei das zweite Decodierungsinformationsdatenpaket (324) patientenbezogene Analyten decodiert.

## Revendications

1. Procédé d'utilisation d'un produit à micro-réseau, le procédé comprenant :
l'obtention d'un produit à micro-réseau (310) ;
l'exécution d'un essai biologique par un premier consommateur utilisant ledit produit à micro-réseau (310) ;
l'obtention, par ledit premier consommateur, à partir d'un fabricant du produit à micro-réseau (310), d'un premier paquet de données d'informations de décodage (324) pour la commande de mesures sur le produit à micro-réseau (310) afin de déterminer si l'essai a été exécuté correctement, mais non pour des données analytiques d'analytes sur le produit à micro-réseau (310) ; et
l'obtention, par un deuxième consommateur, à partir d'un fabricant du produit à micro-réseau (310), d'un deuxième paquet de données d'informations de décodage (324) pour des données analytiques sur le produit à micro-réseau (310).

2. Procédé selon la revendication 1, dans lequel le premier consommateur est un laboratoire ou un laboratoire de services d'analyses et dans lequel le deuxième consommateur est un professionnel de la santé.

3. Procédé selon la revendication 1, dans lequel le premier paquet de données d'informations de décodage pour commander des mesures sur le produit à micro-réseau (310) permet de commander des mesures incluant des essais à blanc, des étalonnages et des mesures d'intensité de rayonnement pour l'étalonnage et la normalisation, et dans lequel le deuxième paquet de données d'informations de décodage (324) décode des analytes concernant des patients.
